# EUROPEAN PATENT APPLICATION

(11) **EP 2 397 146 A1**
(43) Date of publication of application: **21.12.2011**
(21) Application number: 10006082.1
(22) Date of filing: 11.06.2010
(51) Int. Cl.: A61K 35/76, A61P 35/00, A61K 48/00

(54) **Parvovirus for treatment of tumours by intranasal application**

(71) Applicant: Deutsches Krebsforschungszentrum, 69120 Heidelberg (DE)
(72) Inventor: Rommelaere, Jean, 69118 Heidelberg (DE); Kiprijanova, Irina, 69118 Heidelberg (DE); Schlehofer, Jörg, 69181 Leimen (DE); Ayache, Ali, 69120 Heidelberg (DE); Fischer, Manuel, 69151 Neckargemünd (DE); Thomas, Nadja, 67117 Limburgerhof (DE)
(74) Representative: Schüssler, Andrea

(57) **Abstract**

Described is the use of an oncolytic parvovirus for the manufacture of a medicament for the treatment of a tumor and/or tumor metastases, in particular a brain tumor, by intranasal administration. A preferred parvovirus is H1-PV. Intranasal parvotherapy can improve the prognosis of said tumors. Parvovirus H1 infection leads to killing of tumor cells but does not harm normal brain cells. Thus, the intranasal use of a parvovirus like H1, a related virus or vectors based on such viruses offers the chance of tumor specific therapy without severe neurological side effects.

## Description

The present invention relates to the use of a parvovirus for the manufacture of a medicament for the treatment of a tumor and/or tumor metastases, in particular a brain tumor, by intranasal administration.

Rodent parvoviruses are single-stranded DNA viruses possessing intrinsic oncolytic activity, i.e. they preferentially replicate in and kill cancer cells of both murine and human origin. In this regard, oncolytic parvoviruses, in particular rodent parvoviruses represent novel tools for cancer treatment. Though the virus can enter most nontransformed (normal) cells, infection is abortive and fails to produce progeny virions. In contrast, in many malignant cells or in cells transformed with various means, parvovirus infection leads to cell killing which is not observed in the corresponding nontransformed cells, and can be productive. The enhanced permissiveness of transformed cells for parvoviruses was shown to be due to at least in part to changes in factors associated with (i) cell cycling and differentiation and (ii) controlling viral DNA replication and gene expression. Besides specifically killing cancer cells (oncolysis), these parvoviruses also provide danger signals prompting the immune system to eliminate virus-infected tumours. As a consequence of oncolytic events, the innate and adaptive immune systems gain access to tumour antigens, which results in cross-priming and vaccination effects.

Glioblastoma is a devastating disease with only limited treatment possibilities. Evidently, the prognosis for patients requires new therapies. The replication competent oncolytic viruses discussed above are considered promising since they are able to spread through malignant tissues and induce antitumor immune-responses. Among them, rodent parvoviruses appear to be excellent candidates, due to their natural oncotropism, their capacity to infect human cells, the specific toxicity for neoplastic cells, and the low pathogenicity for humans. In a rat model, H1-PV was able to cause a complete regression of established gliomas without any relaps and the viruses proved able to target various human gliomas, thereby efficiently killing these cells irrespective of their acquired resistance towards known death inducers. However, the routes of administration of parvovirus as regards efficiency and convenience are in need of improvement.

Therefore, it is the object of the present invention to provide a route of administration for oncolytic parvoviruses overcoming the draw backs of the prior art.

According to the invention this is achieved by the subject matters defined in the claims. It was surprisingly found that intranasally administered parvoviruses can successfully be used for highly efficient suppression of cerebral gliomas in rats. In the study leading to the present invention, the concept of H-1PV-based virotherapy of glioma by intranasal application of H-1PV was tested for rat (RG-2 cell-derived) gliomas in immunocompetent rat models. Rats were treated with intranasally administered H-1PV. Tumor regression was monitored by magnetic resonance imaging, and oncolysis was proven by histology. Presence of viral DNA and viral capsids were detected in the brain. Virus replication in tumors may contribute to secondary infection by progeny virus to the efficiency of oncolysis. In immunocompetent rats bearing RG-2 derived tumors, a single intranasal application of H-1PV of the virus was sufficient for remission of advanced and even symptomatic intracranial gliomas without damaging normal brain tissue or other organs. Moreover, H-1PV therapy resulted in significantly improved survival (Kaplan-Meier analysis) in the rat glioma models. The results presented in the examples, below, and the innocuousness of H-1PV for humans argue for the use of H-1PV as a powerful means to perform oncolytic therapy of tumors, in particular malignant gliomas by intranasal application of the parvovirus.

To summarize, intranasal parvotherapy according to the present invention is useful for the therapy of tumors, in particular brain tumors, and can improve the prognosis of said tumors. Parvovirus H1 infection leads to killing of tumor cells but does not harm normal brain cells. Thus, the intranasal use of a parvovirus like H1, a related virus or vectors based on such viruses offers the chance of tumor specific therapy without severe neurological side effects. Advantages of intranasal application would facilitate also multiple applications (if required), e.g. by using a spray. In contrast to parenteral applications, intranasal application would not readily induce serum antibodies, and if serum antibodies might be present, intranassaly applied virus would not be readily neutralized. It is conceivable that also brain metastases of tumors of other than glial origin may be reached by the oncolytic parvovirus.

### Brief description of the drawings

Figure 1: Kaplan-Meyer analysis calculating the probability of survival of tumour bearing animals infected (or not) with H-1PV
   Survival rate of RG2 glioma-bearing rats, treated with PBS (continuous line), empty viral capsids (dashed line) or with H-1PV (dotted line). "Time in days" indicates time after tumour cell implantation.
Figure 2 : MR images of three animals, each from one experimental group
   **(A)** (control-PBS) - intracranial RG2 glioma, intranasally treated with PBS at day 10 post tumour implantation;
   **(B)** (control-inactivated virus) - intracranial RG2 glioma, intranasally treated at day 10 after tumour cell implantation, with empty capsids of H-1PV, final concentration 1x10¹⁰ pfu;
   **(C)** (H-1PV) - intracranial RG2 glioma, intranasally treated with H-1PV, final concentration 1 x 10⁸ pfu.
   a,b,c,d - days after tumour cell implantation (a=10; b=15; c=20; d=25) There are no MRI of animals of group A and B presented, since these animals died at day 15 and day 19 post tumour implantation, respectively. The animal from group C survived further for longer than 80 days.

The present invention relates to the use of an oncolytic parvovirus, preferably a rodent parvovirus for the manufacture of a medicament for the treatment of a tumor and/or tumor metastases by intranasal administration.

The term "parvovirus" as used herein comprises wild-type or modified replication competent derivates thereof as well as related viruses or vectors, e.g., expression vectors, based on such viruses or derivatives. A person skilled in the art is familiar with examples thereof. In a preferred embodiment, the vector containing the DNA of a parvovirus is a virus, e.g. an adenovirus, vaccinia virus, an AAV virus or a parvovirus, such as MVM or H-1, a parvovirus being preferred. The vector may also be ,a retrovirus, such as MoMULV, MoMuLV, HaMuSV, MuMTV, RSV or GaLV. Moreover, the parvovirus can be applied as naked DNA or can be coupled to, or embedded within, a liposome surface thus allowing enhanced delivery.

For constructing (expression) vectors which contain parvoviral DNA, it is possible to use general methods known in the art. These methods include, e.g., in vitro recombination techniques, synthetic methods and in vivo recombination methods.

Suitable parvoviruses, derivatives etc. are known to the person skilled in the art.

Preferably, parvoviruses are used that are capable of propagating and spreading through human tumor cells. The term "propagating" as used herein means that the progeny virions of the parvovirus variant show amplification significantly over input levels (i.e. > 5 fold). The term "spreading through" as used herein means that the progeny virions of the parvovirus variant are capable not only of killing primarily infected cell populations, but are able to cause secondary infections of naive cells with particles produced during the first round of infection.

Preferably, the parvovirus used in the present invention is a rodent parvovirus with rat parvovirus being particularly preferred or a related rodent parvovirus. Examples of preferred related rodent parvovirus are LuIII, Mouse minute virus (MMV), Mouse parvovirus (MPV), Rat minute virus (RMV), Rat parvovirus (RPV) or Rat virus (RV). A particularly preferred rat parvovirus is parvovirus H-1 (H-1PV).

In principle, any brain tumor (or possibly also brain metastases of other primary tumor origin) can be cured by intranasal application of a parvovirus_{,} with gliomas, in particular glioblastomas, being preferred.

For intranasal administration, the parvovirus can be combined with suitable pharmaceutical carriers. Suitable pharmaceutical carriers of a type well known in the art and readily commercially available, include phosphate buffered saline (PBS) solutions, water, emulsions such as oil/water emulsions, wetting agents of various types, sterile solutions, etc. Such carriers can be formulated with the parvovirus by conventional formulating methods for administration to the subject at a suitable dose.

The dosage regimen of the parvovirus is readily determinable within the skill of the art, by the attending physician based on patient data, observations and other clinical factors, including for example the patient's size, body surface area, age, sex, the particular virus, vector, cell, etc. to be administered, the time and route of administration, the tumor type, grade, size etc., general health of the patient, and other drugs or therapies to which the patient is being subjected.

Preferably, intranasal administration is a single dose or three doses daily for three days.

Preferably, a single dose contains between 10⁷ and 10¹⁰ pfu. Ranges between 10⁸ and 10⁹ pfu are particularly preferred.

The below example explains the invention in more detail.

### Example

### Treatment of glioma in a rat model using intranasal application of oncolytic parvovirus H-1 (H-1PV)

### (A) Cells

The rat glioblastoma cell line RG-2 (kindly provided by C. Walz, University of Magdeburg, Germany) was grown in DMEM (Sigma-Aldrich Steinheim, Germany) supplemented with 10% FCS (Biochrom KG Berlin, Germany) and 1% antibiotics (penicillin, streptomycin; Gibco, Invitrogen Corporation Karlsruhe, Germany) in a 5% CO₂ humidified atmosphere at 37°C. Exponentially growing cells to be administered to rats were trypsinized and centrifuged (146 g/10 min), and the pellet was resuspended in DMEM without supplements.

### (B) H-1PV production and infection

H-1PV was amplified in human NBK cells and purified on iodexanol gradients as described previously (Di Piazza et al., J.Virol.2007;81:4186-4198). H-1PV was titrated on NBK indicator cells by plaque assay and further used at multiplicities of infections expressed in plaque-forming units (pfu) per cell.

### (C) Animal Experiments

All animal experiments were carried out in accordance with institutional and state guidelines.

### (D) Experimental design

In total, 20 Wistar rats (Charles River) were used for the experiments. Rat glioma cells (RG2; 1 x 10⁴ per animal) were injected stereotactically into the front lobe of one brain hemisphere brain as previously described in detail (Geletneky et al. [2010] Neurooncology, doi:10.1093/neuonc/nog023). Ten days after cell implantation, ten of tumour-bearing rats have been treated with H-1PV by using intranasal application. For intranasal application, animals were shortly anesthetized with isofloran and received 50 µl PBS containing 10⁸ PFU of H-1PV in each nostril. The control animals (n=10) received either PBS in the same volume or inactivated virus solution (empty capsids, 1 x 10¹⁰ pfu). The head was kept reclined and in a lateral position to ensure flow of virus suspension to the roof of the nasal cavity. Infected animals were kept separately from uninfected control rats under isolator conditions. Tumor growth was monitored by MRI every 4-5 days. Health appearance and weight of animals were monitored daily. Animals with either significant neurological symptoms (paralysis, lateropulsion, etc.) or a body weight drop below 75% of the starting value were euthanized according to institutional guidelines.

### (E) Analyses of tissue samples

Two animals from each of the 3 groups of glioma bearing animals (untreated [5 rats], treated with inactivated virus [5 rats], infected with "active" virus [10]) were sacrificed 3 days after infection for analyses. Brain and various organs were removed and shock-frozen at -192°C (liquid nitrogen) after immersion in freezing medium.

### (F) Magnetic Resonance Imaging

The animals were examined in a 2.45-T MRI Scanner ((Bruker, Ettlingen, Germany)) using Tl-weighted imaging before and after injection of 0.4 mL contrast medium (Gadodiamide, Omniscan, Amersham) into the tail vein. Gadodiamide-enhanced T1 imaging was performed 5 minutes after intranasal administration. During MR examination, rats were anesthetized by isoflurane insufflation (initial dose 2.5%, maintenance 1.6%). Tumor volumes were determined using MRIcro software (Rorden and Brett, Behav.Neurol.2000;12:191-200).

### (G) Statistics

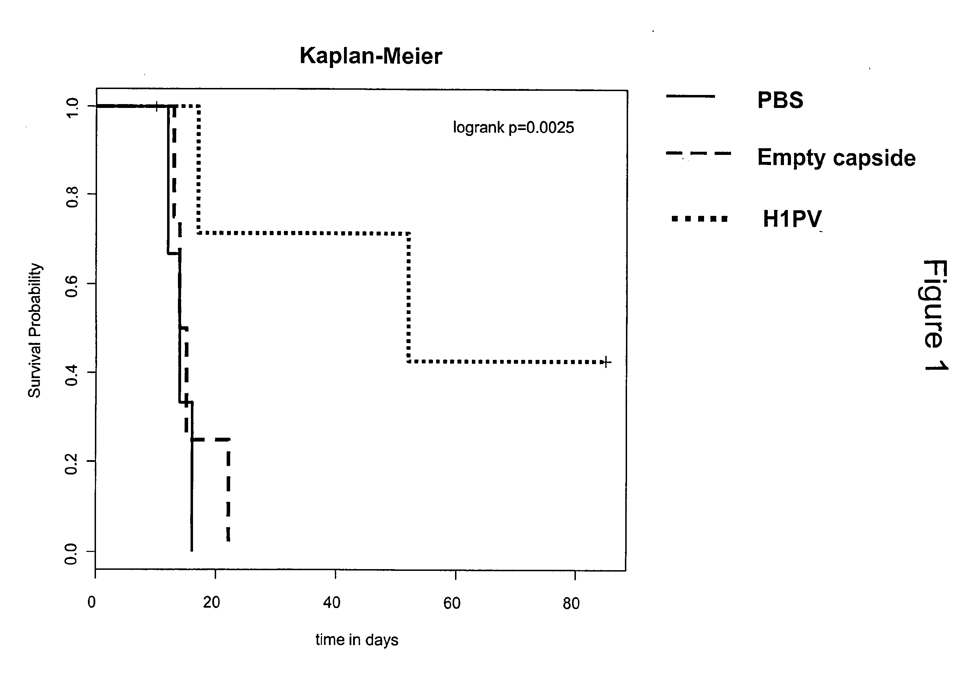

For estimation of survival of tumour-bearing animals infected or not with H-1PV, a Kaplan-Meyer analysis (calculating the probability of survival) was performed (Figure 1).

### (H) Results

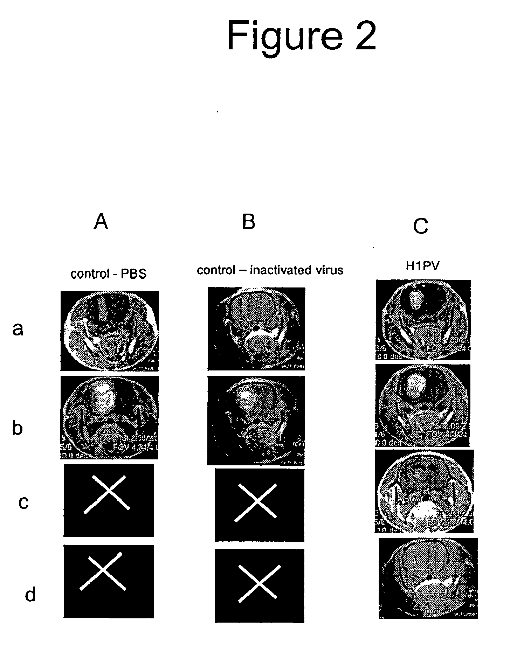

All glioma-bearing rats, treated with intranasal infection of H-1PV, survived significantly longer in comparison to animals of the control groups (Figure 1). Except two animals who died for unknown reasons, MRI of 5 H-1PV-treated rats displayed complete regression of glioma (MRI data), with no obvious side-effects (of those, 2 were killed at day 55 after cell implantation), the remaining 3 animals are still alive (longer than 90 days); see the results presented in Figure 2). Two infected animals were sacrificed for analyses (PCR, immunohistochemistry) 3 days after infection showing the presence of viral DNA in the brain as well as viral capsid proteins. In contrast, all control animals died between two and three weeks after cell implantation with no signs of tumour regression.

## Claims

1. Use of a parvovirus for the manufacture of a medicament for the treatment of a tumor and/or tumor metastases by intranasal administration.

2. A parvovirus for use in a method of treatment of a tumor by intranasal administration.

3. The use according to claim 1 or 2, wherein intranasal administration is a single dose once per day for 3 days.

4. The use according to any one of claims 1 to 3, wherein a single dose contains between 10⁷ and 10¹⁰ pfu.

5. The use according to claim 4, wherein a single dose contains between 10⁸ and 10⁹ pfu.

6. The use according to any one of claims 1 to 5 wherein the parvovirus is a rodent parvovirus.

7. The use according to claim 6, wherein the rodent parvovirus is a rat parvovirus.

8. The use according to claim 7, wherein the rat parvovirus is H1-PV.

9. The use according to any one of claims 1 to 8, wherein the tumor is a brain tumor and/or the metastases are cerebral metastases.

10. The use according to claim 9, wherein the brain tumor is glioma.

11. The use according to claim 10, wherein the glioma is a glioblastoma.
